# EUROPEAN PATENT SPECIFICATION

(11) **EP 2 544 643 B1**
(45) Date of publication and mention of the grant of the patent: **19.09.2018**
(21) Application number: 11715020.1
(22) Date of filing: 11.03.2011
(51) Int. Cl.: A61F 13/02

(54) **VAGINAL PLASTER FOR THE PREVENTION OF FLUID ENTERING INTO THE VAGINA**
VAGINALPFLASTER ZUR VERMEIDUNG VOM EINTRITT VON FLÜSSIGKEIT IN DIE VAGINA
PANSEMENT VAGINAL AFIN D'ÊMPECHER L'INFILTRATION DE LIQUIDE DANS LE VAGIN

(30) Priority: 12.03.2010 HU 1000059 U
(43) Date of publication of application: 16.01.2013
(73) Proprietor: Gynopatch Kft., 1036 Budapest (HU)
(72) Inventor: KOVÁCS, Péter, 1039 Budapest (HU)
(74) Representative: Derzsi, Katalin
(86) International application number: PCT/HU2011/000021
(87) International publication number: WO 2011/110878

(56) References cited:
- US-A- 4 982 450
- US-A- 5 114 419
- US-A1- 2009 198 203

## Description

The present application relates to vaginal plaster for the prevention of entering external fluid into the vagina, above all for the prevention of infections occurring in the course of aquatic sport activities.

Therefore the application relates to a plaster for the prevention of health infection, which closes flexibly the female external genitalia from outside, this way inhibiting the development of the infections of female genitalia obtainable in the course of the usual swimming pool, free water, sauna, solarium, fitness and sport activities conducted in wet environment, most often the medical background of these infections is fungal disease, or infection caused by bacteria and other parasites.

It is known, that in the last years the number of the non-sexually transmitted gynaecological vaginal infections rocketed. The locations of the infections are mostly public swimming pools, free waters, saunas, solariums, fitness salons. The environmental pollution, mass attendance, and the non satisfactory personal hygiene can be in their background.

Mostly different disinfection crèmes, vaginal suppositories and tampons are used for the prevention. But the success of such prevention is incalculable, in many cases the avoidance of the infections is only a matter of luck, since in the course of swimming different amount of water flows into the vagina of many women, mainly for those, who have already given birth to many children. This may lead first to vaginitis, then to uteritis and pelvic inflammation, consecutive infertility.

Method or device, preventing the infection in the course of pursuing water sports, directly blocking the outside female genitalia from the external world, for preventing the disease is not known according to prior art.

The usual pathogens spread in water, and generally in wet environment can be fungi, like *Chlamydia, Trichomonas vaginalis,* and mixed bacteria. The plaster according to the invention prevents them from entering the vagina from water or wet environment.

Development of the present plaster was promoted by the custom recently becoming fashionable, i.e. partly or fully shaving, epilating the hair of the genitalia.

The known solutions representing the background of the present plaster of the prior art are the following.

EP 466 092 discloses a polyvinyl alcohol based medicinal preparation, applicable as a film, serving local treatment of the sexually transmitted diseases, bacteria, viral and fungal infections. The purpose of the medicinal preparation described is not prevention, but treatment of existing infection, and is not suitable for closing the entry of the vagina in wet environment.

In US 5,483,705 a rigid, kidney shaped protective device is described which fits the body, and protects the genital region of the woman against injuries in the course of sports. Its concave part covers the entry of the vagina and the perineum, without touching them. The purpose of the device described is prevention, but its fitting, closing and material doesn't make it suitable for flexible, waterproof closing of the entry of the vagina against microorganisms.

In HU P9501985 Hungarian patent specification a transdermal plaster is described, containing a substance for the prevention and/or treatment of vaginal atrophy, hypogonadism, decreased libido, osteoporosis, urinary incontinence, ovarian and uterine cancer, and the symptoms of menopause, or for contraception. The plaster is suitable for percutaneous and transmucosal delivering of the active ingredient, but the location of putting it to the body, and its form are not defined.

US 2009/198203 A1 describes an absorbent article comprising a shell and an absorbent structure which together correspond to a vaginal plaster.

A device for protecting the genital, urinary and rectal area became known form patent specification US 5 114 419, having a carrier, a film placed thereon and self-adhesive film on its edges. The device has a rather big extent, it may be used as a slip, short, Bermuda-like shape, or it can be used as underwear. At water sports this device is uncomfortable, it inhibits the breathing of the skin, and not esthetical.

Anyway, the fact is that the solution referred above is not suitable for the prevention of bacterial and fungal infections.

Moreover, a dress of swimsuit form is described in GB 2 316 295, aiming at the prevention of incontinence or menstrual inconveniences. This solution is unsuitable for the flexible, waterproof closure of the entry of the vagina, in order to provide protection against microorganisms.

Several solutions are known from the prior art, in which the plaster is coated with a uniform and porous adhesive, and the plaster contains adhesive-free portions. But none of these concerns closure of the anatomical orifice, they can only be used as wound cover impermeable for bacteria. Exact specification relating to their shape, size application field is not known.

Generally it can be said that although different plasters are used in the medicine, but these were not used yet in any form for the flexible closure of the female genitalia in water environment, for the prevention of disease (infection), and a solution serving this purpose hasn't even been developed yet.

The aim of this technical solution is the development of a device suitable for the existing demands, and for the requirements outlined above.

Therefore the aim of the present application is to provide a vaginal plaster preventing the liquid from entering the vagina, primarily for the prevention of the infections occurring in the course of water sports; wherein its carrier has a defined geometric form, and at least at its margin includes a self-adhesive, flexible film, fixed to one side of the carrier, and fitted with a cover fixed to the self-adhesive margin of the flexible film, practically with the same size and form as the carrier, provided with an adhesive material on its inner surface.

The flexible, at least on its margins self adhesive film may have a pad in the middle of its surface. The pad mentioned occasionally contains a sanitary disinfectant.

The geometric form of the carrier with defined shape is suitably oval, circular, rectangle, triangle, trapezoid or polygon.

The material of the carrier and the cover can be paper or plastic, applicable in the healthcare, known *per se.* The criteria to be met are that it must be suitably clean for applying it in the healthcare, and its thickness, rigidity must be suitable for providing the comfort feeling allowing the application. The rigidity requirement is that it must be suitably rigid for unpacking and application.

The adhesive material on the inner side of the cover should also be a usual, skin-friendly material, known *per se.*

The pad is present between the layers of the plaster, its material is hygroscopic, i.e. textile, irregular thread veil or cellulose or fibres, etc. As it has been mentioned, the hygroscopic pad may contain a sanitary disinfectant, also known *per se.*

The self adhesive plastic film is also known *per se,* the requirement is that it must be skin-friendly.

A characteristic feature of the plaster is that it sticks directly to the outside female genitalia, and to the skin surrounding it. In its size it is adjusted to the size of the genitalia of the women of different age and body weight, according to the size of the skin, and it is shaped in such a way, that its middle portion, covering the labia minora, the clitoris, the entry of the vagina, and, depending on its size, even the anus, is adhesive-free, or it is provided with hygroscopic pad and/or disinfecting material on this area. The embodiment in which the anus is covered as well, is not an object of the invention.

The plaster claimed will be described in relation to the figures, for better understanding.
Figures 1 (the illustrated shape of the plaster is not an object of the present application) and 2 present the possible implementation forms of the swimming pool plaster, from above;
   Figure 3 presents the cross-section of the product in the "A" section plane of Figure 1.

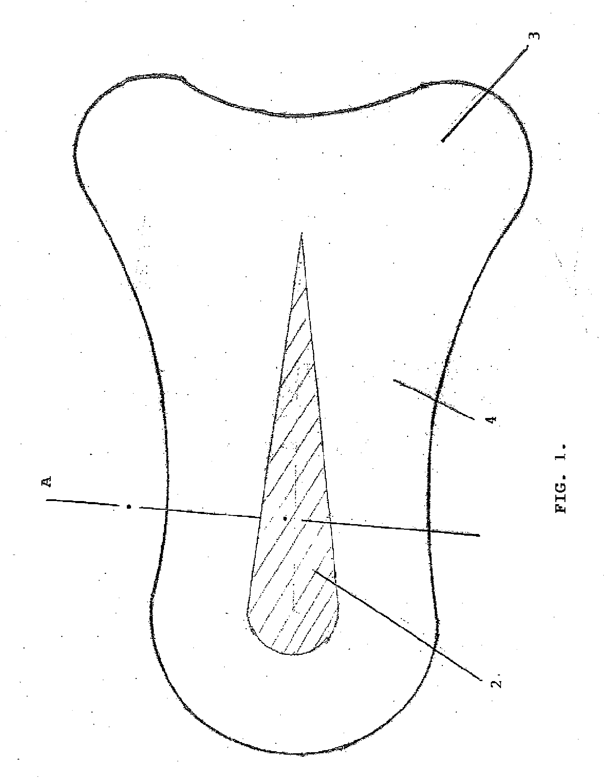

   Figure 1 presents the 1 self adhesive, flexible film (hydrocolloid film), the inner, skin-side part of which sticks, the outer not, located on 4 carrier.
   The 2 pad is on the sticking surface, it doesn't stick to skin.
   The arrows give information for the application (separation of the layers, placement).

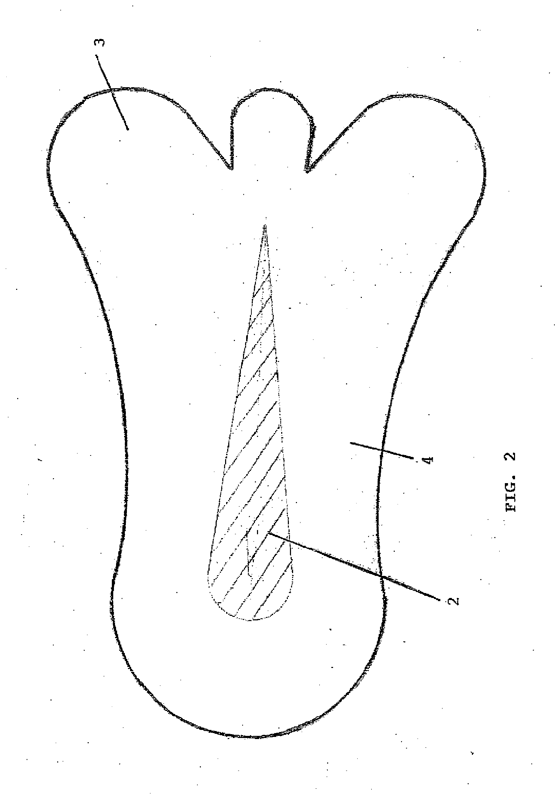

Figure 2 presents also the 1 self adhesive, flexible film and the 2 pad, located on the carrier. But in this case the plaster has 3 loops, to which the 1 self adhesive, flexible film extends to the area towards the anus. This supplement was the result of the experience gained in the course of the experiments, i.e. on the so-called perineum, which is an about 1-2 cm area between the entry of the vagina and the anus, the increased size sticking area of the plaster sticks with greater efficiency, and for a longer time.

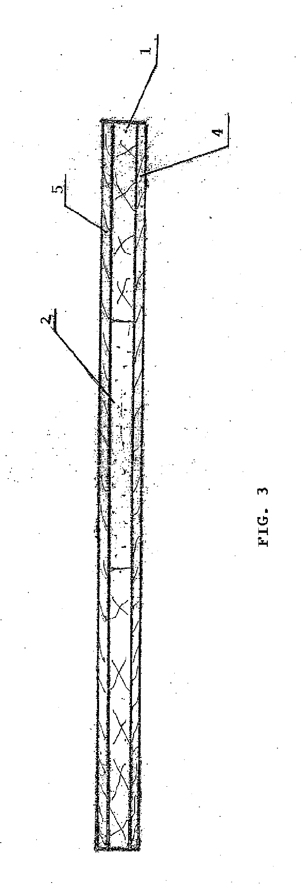

Figure 3 presents the arrangement of the layers according to the above and the cover, in cross-section.

The sticking area is the portion toward the 3 loops of the 1 self adhesive, flexible film. The 2 pad and the 3 loops have paper or other plastic covering. Following the removal of the coverings of the 3 loops the sticky surface of the 1 self adhesive film gets free, therefore it becomes adhesive. Following its sticking, after the removal of the covering of the 2 pad the plaster can be used immediately.

After use the plaster can be simply removed if the skin and the plaster are pulled in opposite directions at the sticking edge.

Numerous experiments were conducted concerning the efficacy of the claimed plaster. These, and the results obtained are demonstrated hereinunder.

### Experiment:

The aim of the experiment was to demonstrate the efficiency of the plaster, i.e. to test whether it really inhibits entry of the most frequently occurring pathogens into the vagina in wet environment.

20 patients were selected, who had recurrent vaginal discharge, and their causes can be attributed to swimming pool infection, in the public bath named by them.

Purity level of the water of the baths and their saunas are regulated by public health regulations. These regulations are obviously respected by the operators, but it is known that at high temperature and in great mass the hygienic quality of the water of the bath changes, and this change is always happening differently. Our experience is that certain kinds of discharges are always ascribed to the same bathes by women suffering from bathing vaginal infection.

The method applied was the following: if the patient suffering from vaginal infection shows up at the gynaecological consulting hours, vaginal smear is taken, and fungal, trichomonas, bacteria, Chlamydia cultivations are carried out, according to the usual microbiological procedures. If the cultivation procedures give positive results, the patient treated with a medicine specific for the pathogen detected. The cultivation procedures mentioned above are repeated, according to the time of cultivation, one week later, and if the results have become negative, half of the volunteered patients (10 persons) have received a plaster according to the application, and the other 10 persons formed a control group, who didn't receive the plaster according to the utility model.

The plaster was used in the same bath or sauna, on the same day, in the same part of the day as in the one where and when they got the infection, according to their opinion. Patients of the control group have also visited the same bath, as the one where they got the infection, according to their opinion.

Considering the incubation time (from the infection until the occurrence of the symptoms) of the pathogens tested, 7 days later the patients were requested to come back for a repeated examination.

Vaginal smear taking and the cultivation examinations were repeated, summary of the results can be seen in Table 1.

Additionally, the subjects were questioned about their experiences with the plaster according to the invention, and a satisfaction formula was filled out both in relation to the use of the plaster, and in relation to the efficiency of the plaster.

**Table 1**

| SUMMARY OF THE RESULTS OF THE CULTIVATION EXPERIMENTS | | | | |
|---|---|---|---|---|
| Level of vaginal infection | | | | |
| | Control group After using the plaster | | Control group Without using the plaster | |
| | Positive | Negative | Positive | Negative |
| Fungi | 1 | 9 | 5 | 5 |
| Trichomonas | 0 | 10 | 3 | 7 |
| Chlamydia | 0 | 10 | 1 | 9 |
| Enteric bacteria | 0 | 10 | 3 | 7 |

Discussion: it can be seen from the table that following the application of the plaster only 1 of 10 persons received fungal infection, nobody had trichomonas, Chlamydia and enteric bacterial infection, while in the control group fungal infection was detected in 5 of 10 persons, Trichomonas infection was detected in 3 patients, Chlamydia infection was detected in 1 patients, and enteric bacterial infection was detected in 3 patients. Based on the results it can be said that the plaster according to the invention can be efficiently used against spreading of microbial infections into the vagina occurring in wet environment.

According to the examinations the plaster met the requirements both in efficiency and in applicability.

## Claims

1. Vagina! plaster having a carrier (4) for the prevention of entering external fluid into the vagina, above all for the prevention of infections occurring in the course of aquatic sport activities; wherein its carrier (4) has a defined geometric form, and at least at its margin includes a self-adhesive, flexible film (1), fixed to one side of the carrier (4), and the flexible film (1) mentioned above is fitted with a cover (5) fixed to the self-adhesive margin of the flexible film (1), practically with the same size and form as the carrier, provided with an adhesive material on its inner surface, its inner surface being suitable for sticking directly to the outside female genitalia and to the skin surrounding it, **characterised in that** the plaster has a pad (2) in the middle part of its surface, corresponding to the labia minora, and has three loops, to which the self adhesive, flexible film (1) extends to the area towards the anus.

2. Plaster according to Claim 1, with a pad (2), containing sanitary disinfectant.

3. Plaster according to any of Claims 1-2, with carrier (4) with oval, circular, rectangle, triangle, trapezoid or polygon geometric form.

4. Plaster according to any of Claims 1-3, with a carrier and a cover made of paper or plastic, applicable in the healthcare.

5. Plaster according to any of Claims 1-4, with a pad (2), made of hygroscopic material.

## Patentansprüche

1. Vaginalpflaster mit einem Träger (4) zur Vermeidung des Eintritts von externem Fluid in die Vagina, vor allem zur Vermeidung von Infektionen, die im Verlauf von Wassersportaktivitäten auftreten, wobei sein Träger (4) eine definierte geometrische Form hat und mindestens an seinem Rand eine selbstklebende flexible Folie (1) aufweist, die an einer Seite des Trägers (4) befestigt ist, wobei die oben erwähnte flexible Folie (1) mit einer Abdeckung (5) versehen ist, die am selbstklebenden Rand der flexiblen Folie (1) befestigt ist und praktisch dieselbe Größe und Form wie der Träger hat und auf ihrer Innenfläche mit einem Klebematerial versehen ist, wobei ihre Innenfläche dazu geeignet ist, an den äußeren weiblichen Genitalien und an der umgebenden Haut zu kleben, **dadurch gekennzeichnet, dass** das Pflaster ein Kissen (2) im mittleren Teil seiner Oberfläche hat, das den kleinen Schamlippen entspricht, sowie drei Schlaufen hat, zu denen sich die selbstklebende flexible Folie (1) zu dem Bereich zum After hin erstreckt.

2. Pflaster nach Anspruch 1, mit einem Kissen (2), das Hygienedesinfektionsmittel enthält,

3. Pflaster nach einem der Ansprüche 1 - 2, mit einem Träger (4) mit ovaler, kreisförmiger, rechteckiger, dreieckiger, trapezförmiger oder polygoner geometrischer Form.

4. Pflaster nach einem der Ansprüche 1 - 3, mit einem Träger und einer aus Papier oder Kunststoff hergestellten Abdeckung, die im Gesundheitsbereich anwendbar ist.

5. Pflaster nach einem der Ansprüche 1 - 4, mit einem aus hygroskopischem Material hergestellten Kissen (2) .

## Revendications

1. Pansement vaginal comportant un support (4) servant à la prévention de la pénétration de fluide extérieur dans le vagin, en particulier la prévention d'infections se produisant au cours d'activités sportives aquatiques ; son support (4) présentant une forme géométrique définie, et comportant, au moins au niveau de son bord, un film (1) souple auto-adhésif, fixé à un côté du support (4), et ledit film souple (1) étant pourvu d'un revêtement (5) fixé au bord auto-adhésif du film souple (1), pratiquement de la même taille et forme que le support, pourvu d'un matériau adhésif sur sa surface intérieure, sa surface intérieure étant appropriée pour être collée directement aux organes génitaux externes féminins et à la peau les entourant, **caractérisé en ce que** le pansement comporte une compresse (2) dans la partie médiane de sa surface, correspondant aux petites lèvres, et comporte trois boucles, jusqu'auxquelles le film (1) souple auto-adhésif s'étend, jusqu'à la région adjacente à l'anus.

2. Pansement selon la revendication 1, comportant une compresse (2), contenant un désinfectant sanitaire.

3. Pansement selon l'une quelconque des revendications 1 et 2, dont le support (4) est de forme géométrique ovale, circulaire, rectangle, triangle, trapézoïdale ou polygonale.

4. Pansement selon l'une quelconque des revendications 1 à 3, comportant un support et un revêtement composés de papier ou de plastique, applicables dans le domaine des soins de santé.

5. Pansement selon l'une quelconque des revendications 1 à 4, comportant une compresse (2), composée de matériau hygroscopique.
